# EUROPEAN PATENT SPECIFICATION

(11) **EP 3 321 939 B1**
(45) Date of publication and mention of the grant of the patent: **21.08.2019**
(21) Application number: 17201682.6
(22) Date of filing: 14.11.2017
(51) Int. Cl.: H01B 3/08, G08B 17/103, H01B 3/00, G01N 33/00, H01B 1/14, H01B 5/14

(54) **FILLER STRUCTURE AND ELECTRONIC DEVICE INCLUDING THE SAME**
FÜLLSTOFFSTRUKTUR UND ELEKTRONISCHE VORRICHTUNG DAMIT
STRUCTURE DE REMPLISSAGE ET DISPOSITIF ÉLECTRONIQUE LE COMPRENANT

(30) Priority: 15.11.2016 KR 20160152242; 17.10.2017 KR 20170134819
(43) Date of publication of application: 16.05.2018
(73) Proprietor: Samsung Electronics Co., Ltd., Gyeonggi-do 16677 (KR)
(72) Inventor: BAE, Minjong, 16678 Gyeonggi-do (KR); KIM, Jinhong, 16678 Gyeonggi-do (KR); KIM, Hajin, 16678 Gyeonggi-do (KR); KOH, Haengdeog, 16678 Gyeonggi-do (KR); KIM, Doyoon, 16678 Gyeonggi-do (KR); KIM, Seyun, 16678 Gyeonggi-do (KR); MIZUSAKI, Soichiro, 16678 Gyeonggi-do (KR); SOHN, Hiesang, 16678 Gyeonggi-do (KR); LEE, Changsoo, 16678 Gyeonggi-do (KR)
(74) Representative: Elkington and Fife LLP

(56) References cited:
- EP-A1- 1 480 233
- WO-A1-94/10098
- GB-A- 2 499 256

## Description

### FIELD OF THE INVENTION

The present disclosure relates to a filler structure and an electronic device including the same.

### BACKGROUND OF THE INVENTION

A composite material is a material that combines two or more different materials and has excellent properties that a single material may not have. Such a composite material is composed of a matrix that is a basic frame and a reinforcing material filled to improve the properties. According to the properties of the reinforcing material, a degree of property improvement of the composite material may be determined. Also, the composite material may be applied to various fields according to a component material thereof.

The composite material may be used as a conductive material or as an insulating material according to a content rate of a conductive filler.

EP 1480233A discloses a resistor having an alumina substrate over which are formed silver electrodes and a resistance body comprising bismuth binder glass.

GB 2499256A discloses a fire detector including a heat radiation source.

### SUMMARY OF THE INVENTION

The invention is defined by the claims.

Provided are filler structures and electronic devices including the same.

Provided are filler structures with enhanced withstanding voltage properties at a high temperature and electronic devices including the same.

Additional aspects will be set forth in part in the description which follows and, in part, will be apparent from the description, or may be learned by practice of the presented embodiments.

According to an aspect of an embodiment, a filler structure includes a substrate, a filler layer spaced apart from the substrate and comprising a matrix material layer and a plurality of conductive fillers, an electrode configured to contact the filler layer and provide an electrical signal to the filler layer, and an insulating layer arranged between the substrate and the electrode, and having an alkali oxide of a content rate of 7% or less.

At least one of the insulating layer and the matrix material layer may include a glass frit.

The glass frit may include an oxide glass frit.

A glass transition temperature of the insulating layer may be 400 °C or higher.

The filler structure further may include a protection layer configured to contact the substrate and prevent the substrate from being oxidized.

At least a part of a material included in the insulating layer may be the same as a material included in the protection layer.

Materials commonly included in the insulating layer and the protection layer may have different content rates.

A coefficient of thermal expansion of the insulating layer may range from about 5 (µm/m/°C) to about 12 (µm/m/°C).

The electrode may be arranged between the filler layer and the substrate.

The filler layer may be arranged between the electrode and the substrate.

The filler layer may include a material that generates heat in response to an electrical signal or have a resistance changing by combination of particles introduced from outside.

The filler layer may include at least one of carbon black, graphite, metal, conductive polymer, metal powder, CNT, oxide, boride, carbide, and chalcogenide.

According to an aspect of another embodiment, an electronic device include a substrate, a filler layer spaced apart from the substrate and comprising a matrix material layer and a plurality of conductive fillers, an electrode configured to contact the filler layer and provide an electrical signal to the filler layer, and an insulating layer arranged between the substrate and the electrode, and having an alkali oxide of a content rate of 7% or less.

At least one of the insulating layer and the matrix material layer may include a glass frit.

The substrate may include a cavity.

The electronic device further may include a protection layer configured to contact the substrate and prevent the substrate from being oxidized.

A content rate of alkali oxide included in the insulating layer may be less than a content rate of alkali oxide included in the protection layer.

The filler layer may emit light by generating heat.

The electronic device further may include a gas chamber through which gas is introduced, and a photodetector configured to detect light passing through the gas chamber in the light emitted by the filler layer.

According to an aspect of another embodiment, an insulating substrate includes a base layer formed of an electrically conductive material, and an insulating layer disposed on the base layer and having an alkali oxide of a content rate of 7% or less.

### BRIEF DESCRIPTION OF THE DRAWINGS

These and/or other aspects will become apparent and more readily appreciated from the following description of the example embodiments, taken in conjunction with the accompanying drawings in which:
FIG. 1 is a cross-sectional view of a filler structure according to an example embodiment;
FIG. 2 is a plan view of the filler structure of FIG. 1 according to an example embodiment;
FIG. 3A is a table of a content rate of a glass material included in a protection layer of FIG. 1 according to an example embodiment;
FIG. 3B is a table of a result of measuring a withstanding voltage when an insulating layer includes the same material as that of a protection layer according to an example embodiment;
FIG. 4A is a table of a composition of an insulating layer having a lower content rate of alkali oxide than FIG. 3A according to an example embodiment;
FIG. 4B is a table of a result of measuring a withstanding voltage by using the insulating layer of FIG. 4A according to an example embodiment;
FIG. 5 is a cross-sectional view of a filler structure according to another example embodiment;
FIG. 6 is a cross-sectional view of a filler structure in which an electrode is arranged in an upper surface of a filler layer according to an example embodiment;
FIG. 7 is a cross-sectional view of a filler structure in which first and second electrodes are arranged with a filler layer therebetween according to an example embodiment;
FIG. 8 shows a heating device including a filler structure according to an example embodiment;
FIG. 9 illustrates an example of a gas sensor including a filler structure; and
FIG. 10 illustrates an example of a substrate having an insulating property.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. Like reference numerals refer to like elements throughout. Expressions such as "at least one of," when preceding a list of elements, modify the entire list of elements and do not modify the individual elements of the list.

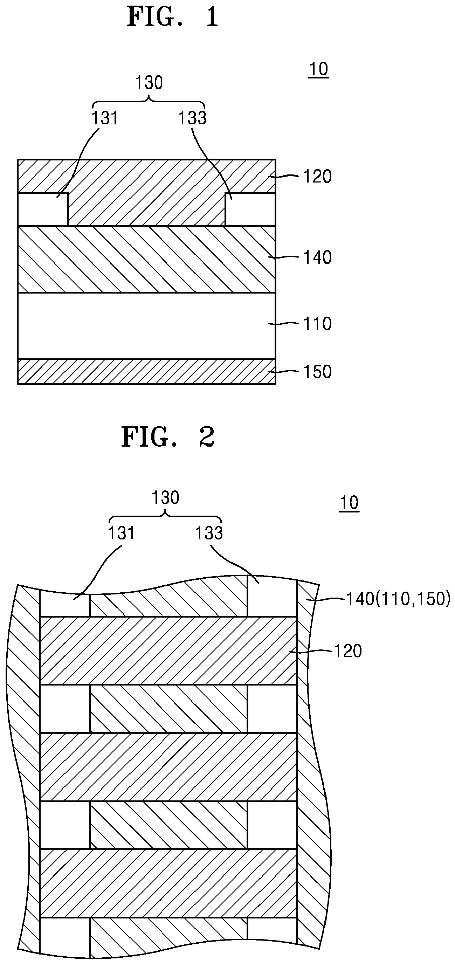

FIG. 1 is a cross-sectional view of a filler structure 10 according to an example embodiment. FIG. 2 is a plan view of the filler structure 10 of FIG. 1 according to an example embodiment.

As shown in FIGS. 1 and 2, the filler structure 10 may include a substrate 110 having electrical conductivity, a filler layer 120 spaced apart from the substrate 110 and including a plurality of conductive fillers, electrodes 130 contacting the filler layer 120 and providing an electrical signal to the filler layer 120, and an insulating layer 140 arranged between the substrate 110 and the electrodes 130 and preventing generation of a leakage current between the substrate 110 and the electrodes 130. The filler structure 10 may further include a protection layer 150 contacting the substrate 10 and preventing the substrate 10 from being oxidized.

The substrate 110 may be a layer supporting the filler structure 10. The substrate 110 may include a material having a high mechanical strength. In general, a conductive metallic material, etc. may have a high mechanical strength. Thus, the substrate 110 may include a conductive material. For example, the substrate 110 may include a steel plate, for example, steel plate porcelain (SPP). Alternatively, the substrate 110 may be formed of a material such as iron (Fe), aluminum (Al), magnesium (Mg), titanium (Ti), zirconium (Zr), zinc (Zn), niobium (Nb), silver (Ag), gold (Au), copper (Cu), or an alloy thereof, but is not limited thereto. The substrate 110 may be formed of an insulating material.

The filler layer 120 may include a matrix material layer and a plurality of fillers having electrical conductivity. The filler layer 120 may generate heat in response to an electrical signal or may have an electrical conductivity, for example, resistance, changing by particles introduced from outside. The electrical conductivity or a heating degree of the filler layer 120 may be adjusted by a content rate of fillers of the electrical conductivity. For example, as the content rate of a plurality of fillers increases, the electrical conductivity of the filler layer 120 may increase. An electrical signal flowing through the filler layer 120 may be different according to a change in the electrical conductivity.

The matrix material layer may include a glass material. For example, according to an example embodiment, the matrix material layer may include a glass frit or enamel powder.

At least some of the plurality of fillers may include a crystalline material. The plurality of fillers may be one-dimensional (1D) fillers or two- dimensional (2D) fillers. In other words, the plurality of fillers may have a 1D or 2D extended structure having a nano-scale. For example, according to an example embodiment, at least one of the plurality of fillers may have a nano-wire shape, nano-rod shape, or a similar shape. The plurality of fillers may not have a specific directionality or orientation and may be randomly arranged. In other words, the plurality of fillers may be arranged in random directions.

Alternatively, at least one of the plurality of fillers may have a nano-sheet shape. The plurality of fillers may have a composition having an electrical conductivity (for example, about 1,250 S/m). However, the electrical conductivity may be less or greater depending on the case.

At least some of the plurality of fillers may be in contact with each other. Among the plurality of fillers, horizontally or vertically adjacent fillers may be in contact with each other. The fillers relatively uniformly distributed in the matrix material layer may be electrically connected to each other. The plurality of fillers may configure a network structure.

The plurality of fillers may be formed of a conductive material. The plurality of fillers may be formed of a material that generates heat in response to an electrical signal or has an electrical conductivity, for example, resistance, changing by particles introduced from outside. For example, the plurality of fillers may include carbon black, graphite, metal, conductive polymer, metal powder, CNT, etc. Alternatively, the plurality of fillers may include at least one or at least two of oxide, boride, carbide, and chalcogenide. The oxide used as the plurality of fillers may include, for example, RuO₂, MnO₂, ReO₂, VO₂, OSO₂, TaO₂, IrO₂, NbO₂, WO₂, GaO₂, MoO₂, InO₂, CrO₂, or RhO₂. The boride used as the plurality of fillers may include, for example, Ta₃B₄, Nb₃B₄, TaB, NbB, V₃B₄, or VB. The carbide used as the plurality of fillers may include, for example, Dy₂C or Ho₂C. The chalcogenide used as the plurality of fillers may include, for example, AuTe₂, PdTe₂, PtTe₂, YTe₃, CuTe₂, NiTe₂, IrTe₂, PrTe₃, NdTe₃, SmTe₃, GdTe₃, TbTe₃, DyTe₃, HoTe₃, ErTe₃, CeTe₃, LaTe₃, TiSe₂, TiTe₂, ZrTe₂, HfTe₂, TaSe₂, TaTe₂, TiS₂, NbS₂, TaS₂, Hf₃Te₂, VSe₂, VTe₂, NbTe₂, LaTe₂, or CeTe₂.

A thickness of each of the plurality of fillers may range from about 1 nm to about 1,000 nm. A size of each of the plurality of fillers may range from 0.1*µ*m to about 500*µ*m. A content rate of each of the plurality of fillers in the filler layer 120 may range from about 0.1 (vol%) to about 100 %(vol%).

The matrix material layer of the filler layer 120 may include a glass frit. The glass frit will be described later.

Alternatively, the matrix material layer may include a heat resisting organic material. For example, according to an example embodiment, the matrix material layer may include an organic polymer. The organic polymer may have a melting temperature of, for example, 200 °C or higher. The organic polymer may include one of polyimide (PI), polyphenylenesulfide (PPS), polybutylene terephthalate (PBT), polyamideimide (PAI), liquid crystalline polymer (LCP), polyethylene terephthalate (PET), polyphenylene sulfide (PPS), and polyetheretherketone (PEEK).

The filler layer 120 may be generally distributed on a surface of the substrate 110. For example, according to an example embodiment, an overlapping region between the filler layer 120 and the substrate 110 may be about 70% or more of the surface of the substrate 110, which may be referred to as planar type heating as heat may be generally generated on the substrate 110.

The plurality of filler layers 120 may be arranged on the substrate 110. The plurality of filler layers 120 may be one-dimensionally or two-dimensionally spaced on the substrate 110. In FIG. 2, the plurality of filler layers 120 are one-dimensionally arranged. Alternatively, at least two of the plurality of filler layers 120 may be connected to each other. The plurality of filler layers 120 have small spaces therebetween, thereby preventing the plurality of filler layers 120 from being bent due to a thermal expansion although the plurality of filler layers 120 expand due to heat.

Alternatively, just one filler layer 120 may be provided. For example, according to an example embodiment, the filler layer 120 may be arranged in a center region of the substrate 110. When only one filler layer 120 is provided, the filler layer 120 may include one or more openings. Thus, even though the filler layer 120 expands due to heat, a shape thereof may not be deformed.

The electrodes 130 may be adjacent to the filler layer 120. The electrodes 130 may include a first electrode 131 contacting one region of the filler layer 120 and a second electrode 133 contacting another region of the filler layer 120. A pair of electrodes 130 may be arranged for each of the filler layers 120. Thus, the electrodes 130 may provide electrical signals to the filler layers 120, and the filler layers 120 may generate heat according to the electrical signals.

The electrodes 130 may include a material having an excellent electrical conductivity. The first electrode 131 and the second electrode 133 may include at least one of Ag, Al, indium tin oxide (ITO), Cu, Mo, and Pt but are not limited thereto.

Meanwhile, the insulating layer 140 may be arranged between the electrodes 130 and the substrate 110 to prevent a leakage current from being generated between the substrate 110 and the electrodes 130 or between the substrate 110 and the filler layer 120. The insulating layer 140 may include a composition that is well bonded to the filler layer 120. The insulating layer 140 may include a glass frit. For example, the insulating layer 140 may include an oxide glass frit, for example, at least one of B₂O₃, Na₂O, ZnO, Al₂O₃, BaO, P₂O₅, CuO, TiO₂, ZrO₂, BiO₂, NiO, and CoO.

The insulating layer 140 according to an embodiment may include a glass frit and may not include an alkali oxide or may include a low content rate of an alkali oxide, so as to have a high glass transition temperature with good adhesion to adjacent layers. For example, the insulating layer 140 may include an alkali oxide of a content rate of 7% or less.

The insulating layer 140 may have a glass transition temperature Tg of 400 °C or higher. The glass transition temperature may be a value indicating heat resistance and may be measured using thermomechanical analysis (TMA), dynamic mechanical analysis (DMA) or the like. If the insulating layer 140 has the glass transition temperature Tg of 400 °C or higher, the insulating layer 140 may have excellent oxidation resistance and may efficiently block flow current even at a high temperature (400 °C or higher), thereby stably securing an insulating property.

Alkali metal components have a very small radius of cation (for example, Li+, Na+, K+) and a low electrovalence. If the insulating layer 140 includes a large amount of alkali oxide, a lot of electrical conductive paths are generated. As a result, a discharge is generated in the insulating layer 140, and thus a material in the insulating layer 140 is broken, thereby causing a thermal breakdown in which the insulating property is lost. A representative material where this phenomenon occurs may be enamel. The enamel has an alkali metal having a content rate of about 11% by weight or more, and thus as a temperature increases, a leakage current increases. As a result, the insulating property is lost at a temperature of about 200 °C or higher.

Thus, the insulating layer 140 according to an embodiment may effectively block the current flow even at the high temperature (for example, 400 °C or higher) by including no alkali oxide or including a small amount of alkali oxide. That is, the insulating layer 140 may have excellent insulation properties and may secure electrical stability. The alkali oxide may include Li2O, Na2O, K2O, and the like. The insulating layer 140 may include an alkali oxide of a content rate of 7% or less so as to maintain the insulating property at 400 °C or higher, but is not limited thereto. The content rate of the alkali oxide may vary depending on a temperature range of the high temperature. That is, the insulating layer 140 may include the alkali oxide of a content rate of 7% or less in order to maintain the insulating property at 700 °C or higher.

The insulating layer 140 may have a thickness of 100 *µ*m to 300 *µ*m. The insulating layer 140 may have a thickness of, for example, 100 *µ*m to 280 *µ*m, for example, a thickness of 100 *µ*m to 250 *µ*m, for example, a thickness of 100 *µ*m to 230 *µ*m, and for example, a thickness of 100 *µ*m to 180 *µ*m. If the thickness of the insulating layer 140 is less than the above range, an insulating effect may be low and there may be damage by an external impact. Conversely, if the thickness exceeds the above range, the cost may increase but heating efficiency may be reduced, and thus the insulating layer 140 may be appropriately used within the above range. The insulating layer 140 may be a single layer or a plurality of layers as the case may be.

The protection layer 150 covering the substrate 110 may be arranged in order to prevent the substrate 110 from being exposed to the outside and oxidized. The insulating layer 140 and the protection layer 150 may include the glass material, for example, the glass frit, like the filler layer 120.

Coefficients of thermal expansion of the substrate 110, the filler layer 120, the insulating layer 140, and the protection layer 150 may range from about 5 (µm/m/°C) to about 12 (µm/m/°C). Alternatively, coefficients of thermal expansion of the substrate 110, the filler layer 120, the insulating layer 140, and the protection layer 150 may range from about 9 (µm/m/°C) to about 11 (µm/m/°C). The substrate 110, the filler layer 120, the insulating layer 140, and the protection layer 150 may have similar coefficients of thermal expansion. This is because if the coefficients of thermal expansion between the layers are similar, stress due to a thermal deformation may be small.

The substrate 110 may include the steel plate having a high mechanical strength in order to maintain an outer shape of a device, whereas all the filler layer 120, the insulating layer 140, and the protection layer 150 may have a glass frit in order to have a high bonding property with the substrate 110 or therebetween and a similar coefficient of thermal expansion.

That is, each of the filler layer 120, the insulating layer 140, and the protection layer 150 may include the glass frit to increase adhesion therebetween. The glass frit may include an oxide glass frit. For example, the glass frit may include one or more oxides selected from the group consisting of silicon oxide, lithium oxide, nickel oxide, cobalt oxide, boron oxide, potassium oxide, aluminum oxide, titanium oxide, manganese oxide, copper oxide, zirconium oxide, phosphorus oxide, zinc oxide, bismuth oxide, argon oxide, lead oxide, and sodium oxide.

The glass frit may include oxide glass frit having an additive added thereto. The additive may include at least one of lithium (Li), nickel (Ni), cobalt (Co), boron (B), potassium (K), aluminum (Al), titanium (Ti), manganese (Mn), copper (Cu), zirconium (Zr), phosphorus (P), zinc (Zn), bismuth (Bi), lead (Pb), and sodium (Na) but is not limited thereto.

At least some of the materials included in the insulating layer 140 may be the same as the materials included in the protection layer 150. Alternatively, the protection layer 150 may include all materials included in the insulating layer 140. Although the insulating layer 140 and the protection layer 150 include the same material, since the insulating layer 140 is to prevent the leakage current from being generated between the electrodes 130 and the substrate 110, and the protection layer 150 is to prevent the substrate 110 from being oxidized, a content rate may be different. For example, according to an example embodiment, a content rate of alkali oxide included in the insulating layer 140 may be less than a content rate of alkali oxide included in the protection layer 150. The content rate of alkali oxide included in the insulating layer 140 may be 7 % or less. The content rate of alkali oxide included in the protection layer 150 may exceed 7%. Alternatively, the insulating layer 140 may not include alkali oxide, and the protection layer 150 may include alkali oxide.

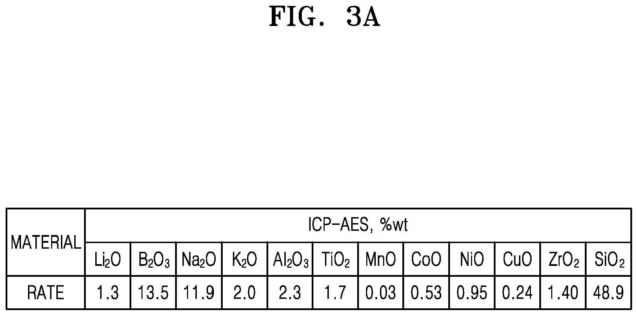

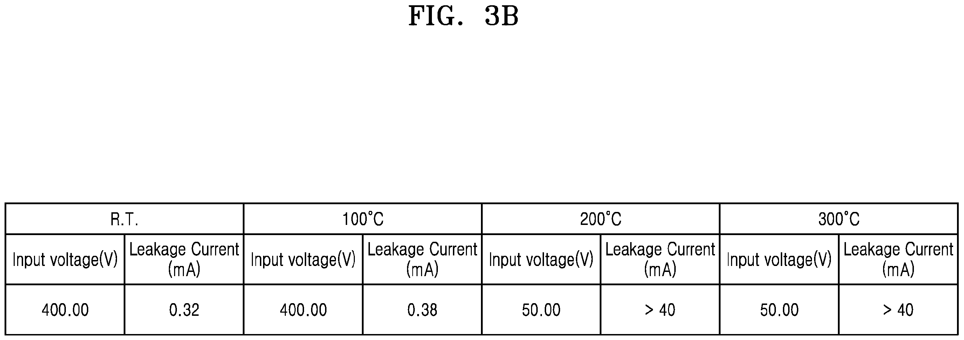

FIG. 3A is a table of a content rate of a glass frit included in a protection layer of FIG. 1 according to an example embodiment. FIG. 3B is a table of a result of measuring a withstanding voltage when an insulating layer includes the same material as that of a protection layer according to an example embodiment. As shown in FIG. 3A, the protection layer may include at least one of LiO, B₂O₃. Na₂O, K₂O, Al₂O₃, TiO₂, MnO, CoO, NiO, CuO, ZrO₂, and SiO₂. The insulating layer including the same material as that of the protection layer may have a thickness of about 150 *µ*m and may be formed between a substrate and an electrode. A leakage current of the insulating layer is measured with respect to temperatures and input voltages. That is, the leakage current with respect to the input voltages and the temperatures is the result of FIG. 3B. As shown in FIG. 3B, the insulating layer generates a leakage current of about 40 mA at a small input voltage of about 50 V at a temperature of about 200 °C. Thus, the protection layer including at least one of LiO, B₂O₃. Na₂O, K₂O, Al₂O₃, TiO₂, MnO, CoO, NiO, CuO, ZrO₂, and SiO₂ may have a low withstanding voltage at a high temperature.

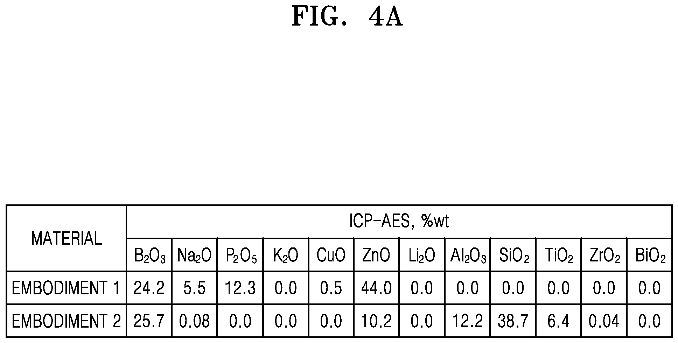

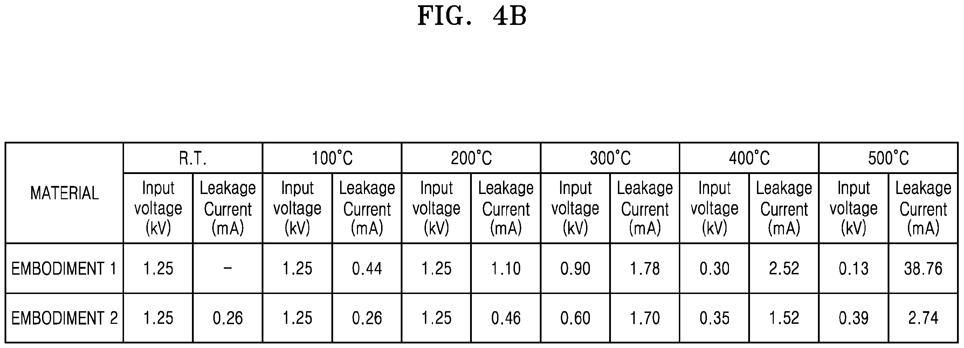

FIG. 4A is a table of a composition of an insulating layer having a lower content rate of alkali oxide than FIG. 3A according to an example embodiment. FIG. 4B is a table of a result of measuring a withstanding voltage by using the insulating layer of FIG. 4A according to an example embodiment.

As shown in FIG. 4A, insulating layers of Embodiments 1 and 2 have no alkali oxide of Li₂O and K₂O or have a low content rate of alkali oxide of Na₂O. In Embodiment 1, a content rate of Na₂O may be about 5.5 %. In Embodiment 2, a content rate of Na₂O may be about 0.08 %. The insulating layers having the content rate of the material and a thickness of about 150 *µ*m are formed and then leakage currents thereof are measured with respect to temperatures and input voltages. As a result, as shown in FIG. 4B, at a high input voltage and a high temperature, the insulating layers generate no leakage current or a minor amount of leakage current, and at a high temperature of about 500 °C, generate a leakage current less than about 40 mA.

According to a type and a content rate of a glass frit, the glass frit may be weak or not effective to prevent a leakage current from being generated. Thus, the insulating layer may include a glass frit different from that of a protection layer or may have a content rate different from that of the protection layer even if the insulating layer includes the same material as that of the protection layer. In particular, the insulating layer may have a low or no content rate of alkali oxide with respect to the glass frit than the protection layer, and thus a high temperature withstanding voltage property of a filler structure may be enhanced. If the insulating layer has alkali oxide of about 70 % or less, the high temperature withstanding voltage property of a filler structure may be enhanced.

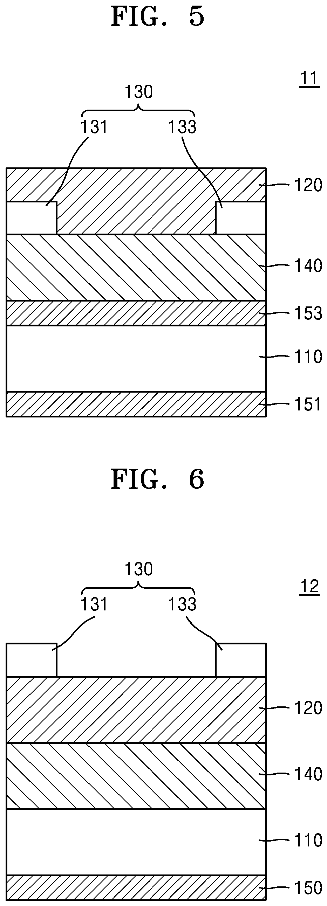

FIG. 5 is a cross-sectional view of a filler structure 11 according to another example embodiment. Upon comparing FIGS. 1 and 5, the filler structure 11 according of FIG. 5 may further include a protection layer 153 contacting the substrate 110 between the substrate 110 and the insulating layer 140. A protection layer commonly arranged in FIGS. 1 and 5 may be a first protection layer 151, and a protection layer additionally arranged in FIG. 5 may be a second protection layer 153. The second protection layer 153 may include the same material as that of the first protection layer 151.

Material components of the first protection layer 151 and the substrate 110 for well bonding of the first protection layer 151 and the substrate 110 may be well known. The second protection layer 153 including the same material as that of the first protection layer 151 may be coated on an upper surface of the substrate 110 and then the insulating layer 140 may be arranged on the second protection layer 160, and thus a bonding force of the insulating layer 140 may be increased because the insulating layer 140 may include a glass material like the second protection layer 153 except for a small difference in a content rate and a type of the glass material. Thus, sequential bonding of the insulating layer 140, the second protection layer 153, and the substrate 110 may have a higher bonding force than that of direct bonding of the insulating layer 140 and the substrate 110.

FIG. 6 is a cross-sectional view of a filler structure 12 in which an electrode is arranged in an upper surface of a filler layer, according to an example embodiment.

Upon comparing FIGS. 1 and 6, the electrodes 130, the filler layer 120, the insulating layer 140, the substrate 110, and the protection layer 150 may be sequentially arranged in the filler structure 12 of FIG. 6. The filler layer 120 and the insulating layer 140 may be arranged between the electrodes 130 and the substrate 110, and thus a space between the electrodes 130 and the substrate 110 may be increased. Thus, a possibility that a leakage current is generated between the electrodes 130 and the substrate 110 may be reduced.

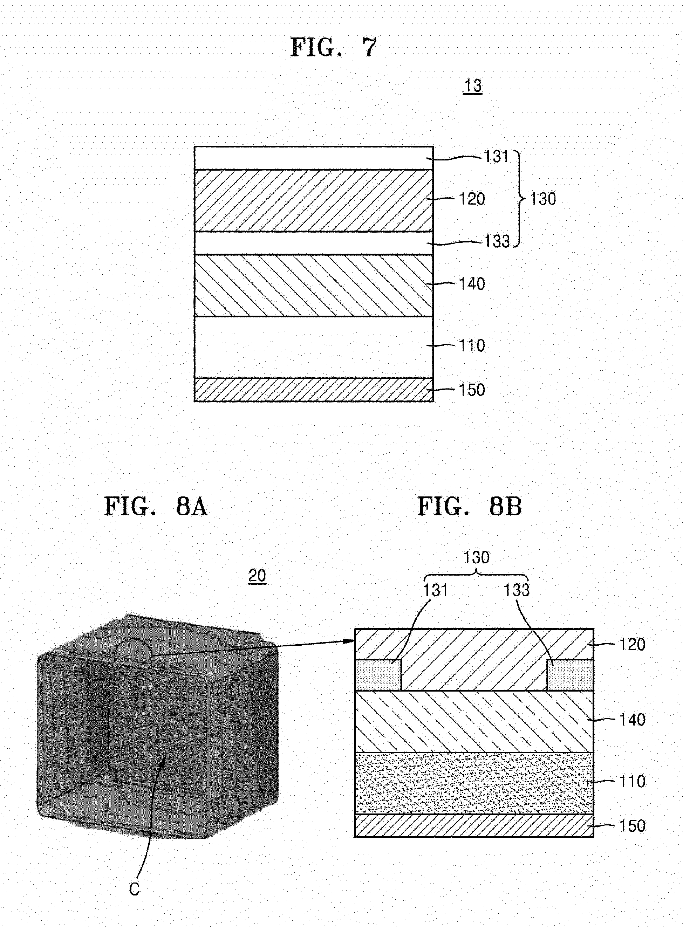

FIG. 7 is a cross-sectional view of a filler structure 13 in which first and second electrodes are arranged with a filler layer therebetween, according to an example embodiment. Upon comparing FIGS. 1 and 7, the first electrode 131 of the filler structure 13 of FIG. 7 may be arranged in an upper surface of the filler layer 120, and the second electrode 133 thereof may be arranged in a lower surface thereof. The filler layer 120 of FIG. 7 may be a single layer. Alternatively, the filler layer 120 may include one or more openings. The same material as that of the insulating layer 140 may be arranged in the openings so that the first electrode 131 and the second electrode 133 may be spaced apart from each other.

FIG. 8 shows an electronic device 20 including a filler structure according to an example embodiment. As shown in FIG. 8, the electronic device 20 may be an electric oven. The electronic device 20 may form a cavity C. The electronic device 20 may have the cavity C that includes five surfaces, has a hexahedron shape, and inwardly opens forward. Thus, food that is a heating target may be placed in the cavity C. Although the electronic device 20 of FIG. 8 has the filler structure 10 of FIG. 1, at least one surface of the electronic device 20 may include the above-described filler structures 10, 11, 12, and 13. For example, according to an example embodiment, an upper surface of the electronic device 20 may include the above-described filler structures 10, 11, 12, and 13. However, the present disclosure is not limited thereto. The above-described filler structures 10, 11, 12, and 13 may be included in at least two surfaces, upper and lower surfaces, or both sides of the electronic device 20.

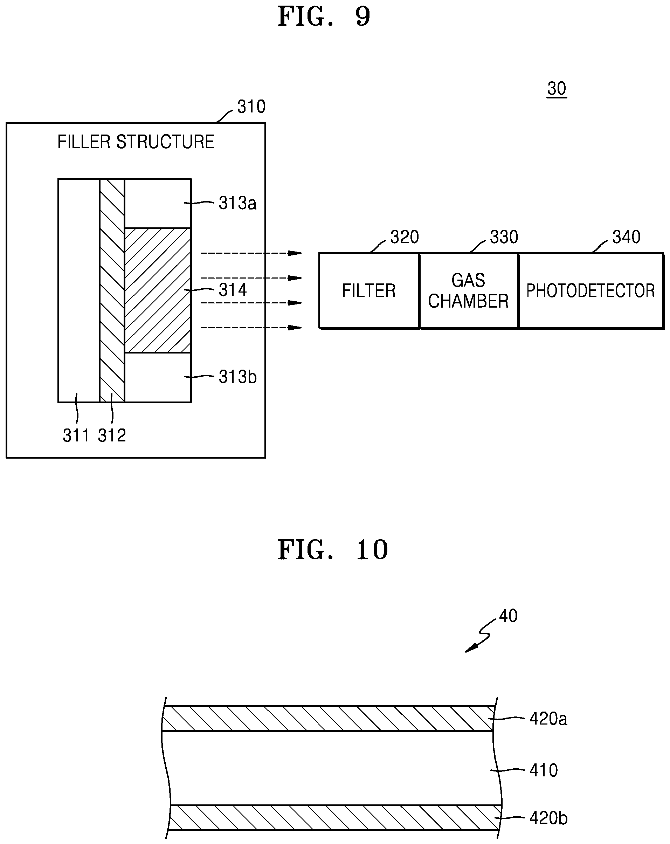

The filler structure according to an embodiment may be applied to a gas sensor which is a kind of an electronic device. FIG. 9 illustrates an example of a gas sensor 30 including a filler structure 310. The gas sensor 30 may be a gas sensor that detects gas using light. As shown in FIG. 9, the gas sensor 30 may include the filler structure 310, a filter 320, a gas chamber 330, and a photodetector 340.

The filler structure 310 may emit specific light such as infrared rays while generating heat and may include a substrate 311, an insulating layer 312, electrodes 313a and 313b, and a filler layer 314,. The substrate 311 and the electrodes 313a and 313b shown in FIG. 9 may be formed of the same material as the substrate 110 and the electrode 130 shown in FIG. 1, but are not limited thereto. The substrate 311 and the electrodes 313a and 313b shown in FIG. 9 may be formed of a material more suitable for the gas sensor 30. For example, the substrate 311 may not be a conductive material. For example, the substrate 311 may include any one of silica glass, quartz glass, polyimide, glass fiber, ceramics, etc., and the electrodes 313a and 313b may be made of silver-palladium (Ag-Pd) alloy, molybdenum (Mo), tungsten (W), platinum (Pt), and the like.

The insulating layer 312 may be formed of the same material as the insulating layer 140 described with reference to FIG. 1. For example, the insulating layer 312 may be formed of a material that is well adhered to adjacent layers, e.g., the substrate 311, the electrodes 313a and 313b, and the filler layer 314, and has excellent withstand voltage properties at a high temperature. The insulating layer 312 may include a glass frit and may not include an alkali oxide or may include a lower content rate of alkali oxide. For example, the insulating layer 312 may include an alkali oxide of a content rate of 7% or less. The insulating layer 312 may have the glass transition temperature Tg of 400 °C or higher.

Also, the filler layer 314 may include a material that emits light, e.g., infrared rays, by generating heat. For example, the filler layer 314 may includes at least one of indium tin oxide (ITO), indium zinc oxide (IZO), zinc oxide (ZnO), tin oxide (SnO₂), antimony-doped tin oxide (ATO), Al-doped zinc oxide (AZO), gallium-doped zinc oxide (GZO), TiO₂, and fluorine-doped tin oxide (FTO).

The filter 320 may selectively pass light having a wavelength within a certain range of the light emitted from the filler structure 310. The gas chamber 330 may include a gas inlet (not shown) through which gas is introduced from outside and a gas outlet (not shown) through which the gas is discharged, and may be formed of a material through which the light having passed through the filter 220 passes. The photodetector 340 may detect the light passing through the gas chamber 130. The photodetector 340 may detect an amount of gas in the gas chamber 330 from the detected light. The filler structure according to an embodiment may also be applied to the gas sensor 30 as described above. The filler structure applied to the gas sensor may be heated in response to an electrical signal, but it is not limited thereto. Resistance of the filler structure applied to the gas sensor may change by particles, for example, gas, introduced from outside. A magnitude of the electrical signal received in an electrode may vary due to the change in the resistance corresponding to the gas. A presence or an absence of gas, an amount of gas, and the like may be measured based on the received electrical signal.

In addition, the filler structure may be used in various applications requiring an insulation property, for example, in a defrosting heater of a refrigerator, a heat exchanger, a heating tool, tempered glass, a fuel cell, and a sealing material of a solar cell, etc.

In another embodiment, the filler structure disclosed above may be applied to means or a device that provides warmth to a user. For example, the filler structure may be applied to a hot pack, and may be provided with clothes (such as a jacket or a vest, etc.), gloves, shoes, etc. that the user may wear on the body. At this time, the filler structure may be provided inside the clothes or on an inner surface of the clothes.

In another embodiment, the filler structure may be applied to a wearable device. The filler structure may also be applied to outdoor equipment, which may be applied to a device that emits heat in cold environments.

On the other hand, the insulating layer having a low content rate of alkali oxide described above is not limited to the filler structure. The insulating layer may be applied to various devices to prevent insulation breakdown at a high temperature. The insulating layer according to an embodiment may be disposed on a functional layer that performs a specific function by an external signal, for example, electrical or optical properties inherent to an electrical signal. Here, the electrical property may represent a dielectric constant, a dielectric dissipation factor, a dielectric strength, a resistivity, an electrical conductivity, etc. The optical property may indicate a reflectance, a refractive index, etc. The filler layer described above may have a high electrical conductivity as an inherent electrical property, and thus is an example of a functional layer in which heat is generated in response to an electrical signal. The functional layer may be a heat absorbing layer, a refractive index changing layer, or a reflectance changing layer, in addition to the filler layer. That is, the insulating layer according to an embodiment may be applied to various devices by being disposed on the functional layer.

Alternatively, the insulating layer according to an embodiment may be applied to a substrate of an electronic device that is manufactured at a high temperature or is operable at a high temperature. FIG. 10 illustrates an example of a substrate 40 having an insulating property. A substrate with a high mechanical strength is highly applicable to an electronic device. In general, a highly conductive metal material has a high mechanical strength. However, it is difficult to design a circuit board or the like on a metal substrate due to an electrical conductivity of the metal material. Thus, the substrate 40 according to an embodiment may have the insulating property by disposing insulating layers 420a and 420b on a base layer 410 of a material having a high mechanical strength of electrical conductivity.

As shown in FIG. 10, the substrate 40 having the insulating property may include the base layer 410 formed of an electrically conductive material and the insulating layers 420a and 420b electrically insulating the base layer 410. The insulating layers 420a and 420b may be disposed on both sides of the base layer 410, for example, on an upper surface and a lower surface, but are not limited thereto.The insulating layers 420a and 420b may be disposed on only some surfaces of the base layer 410.

The base layer 410 having the electrical conductivity may be the same material as the substrate 110 shown in FIG. 1, but is not limited thereto.

The insulating layers 420a and 420b may be formed of the same material as the insulating layer 140 described with reference to FIG. 1. For example, the insulating layers 420a and 420b may be formed of a material that is well adhered to the base layer 410 and has excellent withstand voltage properties at high temperatures. The insulating layers 420a and 420b may include a glass frit and may not include an alkali oxide or may include a low content rate of alkali oxide. For example, the insulating layers 420a and 420b may include an alkali oxide of a content rate of 7% or less. The insulating layers 420a and 420b may have the glass transition temperature Tg of 400 °C or higher.

The substrate 40 having the above-described insulation property may be used as a substrate of a semiconductor device, a photoelectric conversion element, and a thin film solar cell, and may have, for example, a flat plate shape. A shape and a size of the substrate 40 may be appropriately determined according to sizes of the semiconductor device, a light emitting device, an electronic circuit, the photoelectric conversion device, and the thin film solar cell to be used. When the substrate 40 is used for the thin film solar cell, the substrate 40 may be, for example, a rectangular shape with one side longer than 1 m.

It should be understood that the example embodiments described herein should be considered in a descriptive sense only and not for purposes of limitation. Descriptions of features or aspects within each example embodiment should typically be considered as available for other similar features or aspects in other example embodiments.

While one or more example embodiments have been described with reference to the figures, it will be understood by those of ordinary skill in the art that various changes in form and details may be made therein without departing from the scope as defined by the following claims.

## Claims

1. A filler structure comprising:
a substrate (110);
a filler layer (120) spaced apart from the substrate, comprising a matrix material layer and a plurality of conductive fillers and configured to generate heat in response to an electrical signal or have a resistance changing by combination of particles introduced from outside;
an electrode (130) configured to contact the filler layer and provide an electrical signal to the filler layer;
and an insulating layer (140) arranged between the substrate and the electrode, having an alkali oxide of a content rate of 7% or less and configured to prevent a leakage current from being generated between the substrate (110) and the electrodes (130) or between the substrate (110) and the filler layer (120).

2. The filler structure of claim 1, wherein at least one of the insulating layer and the matrix material layer comprises a glass frit.

3. The filler structure of claim 2, wherein the glass frit comprises an oxide glass frit.

4. The filler structure of claim 2 or 3, wherein a glass transition temperature of the insulating layer is 400 °C or higher.

5. The filler structure of any preceding claim, further comprising: a protection layer (150) configured to contact the substrate (110) and prevent the substrate from being oxidized.

6. The filler structure of claim 5, wherein at least a part of a material included in the insulating layer (140) is the same as a material included in the protection layer (150), and optionally wherein materials commonly included in the insulating layer and the protection layer have different content rates.

7. The filler structure of any preceding claim, wherein a coefficient of thermal expansion of the insulating layer ranges from 5 (µm/m/°C) to 12 (µm/m/°C).

8. The filler structure of any preceding claim, wherein the electrode (130) is arranged between the filler layer and the substrate.

9. The filler structure of any preceding claim, wherein the filler layer (120) is arranged between the electrode (130) and the substrate (110).

10. The filler structure of any preceding claim, wherein the filler layer (120) comprises a material that generates heat in response to an electrical signal or has a resistance changing by combination of particles introduced from outside, and optionally wherein the filler layer comprises at least one of carbon black, graphite, metal, conductive polymer, metal powder, CNT, oxide, boride, carbide, and chalcogenide.

11. An electronic device (20, 30, 40) comprising a filler structure according to any preceding claim.

12. The electronic device of claim 11, wherein the substrate comprises a cavity (C).

13. The electronic device of claim 11 or 12, wherein a content rate of alkali oxide included in the insulating layer (140) is less than a content rate of alkali oxide included in the protection layer (150).

14. The electronic device (30) of any of claims 11 to 13, wherein the filler layer (314) emits light by generating heat.

15. The electronic device (30) of claim 14, further comprising:
a gas chamber (330) through which gas is introduced; and
a photodetector (340) configured to detect light passing through the gas chamber in the light emitted by the filler layer.

## Patentansprüche

1. Füllstoffstruktur, umfassend:
ein Substrat (110),
eine vom Substrat beabstandete Füllstoffschicht (120), die eine Matrixmaterialschicht und mehrere leitfähige Füllstoffe umfasst und konfiguriert ist, um Wärme als Reaktion auf ein elektrisches Signal zu erzeugen oder einen sich durch Kombination von Partikeln, die von außen eingeführt werden, ändernden Widerstand aufzuweisen;
eine Elektrode (130), die konfiguriert ist, um die Füllstoffschicht zu kontaktieren und der Füllstoffschicht ein elektrisches Signal bereitzustellen;
und eine zwischen dem Substrat und der Elektrode angeordnete Isolierschicht (140), die ein Alkalioxid in einem Inhaltsanteil von 7% oder weniger aufweist und konfiguriert ist, um zu verhindern, dass ein Kriechstrom zwischen dem Substrat (110) und den Elektroden (130) oder zwischen dem Substrat (110) und der Füllstoffschicht (120) erzeugt wird.

2. Füllstoffstruktur nach Anspruch 1, wobei mindestens eine der Isolierschicht und der Matrixmaterialschicht eine Glasfritte umfasst.

3. Füllstoffstruktur nach Anspruch 2, wobei die Glasfritte eine Oxidglasfritte umfasst.

4. Füllstoffstruktur nach Anspruch 2 oder 3, wobei eine Glasübergangstemperatur der Isolierschicht 400°C oder mehr beträgt.

5. Füllstoffstruktur nach einem der vorhergehenden Ansprüche, ferner umfassend: eine Schutzschicht (150), die konfiguriert ist, um das Substrat (110) zu kontaktieren und zu verhindern, dass das Substrat oxidiert wird.

6. Füllstoffstruktur nach Anspruch 5, wobei zumindest ein Teil eines in der Isolierschicht (140) enthaltenen Materials das gleiche ist wie ein in der Schutzschicht (150) enthaltenes Material, und optional wobei Materialien, die üblicherweise in der Isolierschicht und der Schutzschicht enthalten sind, unterschiedliche Inhaltsanteile aufweisen.

7. Füllstoffstruktur nach einem der vorhergehenden Ansprüche, wobei ein Wärmeausdehnungskoeffizient der Isolierschicht im Bereich von 5 (µm/m/°C) bis 12 (µm/m/°C) liegt.

8. Füllstoffstruktur nach einem der vorhergehenden Ansprüche, wobei die Elektrode (130) zwischen dem Füllstoffschicht und dem Substrat angeordnet ist.

9. Füllstoffstruktur nach einem der vorhergehenden Ansprüche, wobei die Füllstoffschicht (120) zwischen der Elektrode (130) und dem Substrat (110) angeordnet ist.

10. Füllstoffstruktur nach einem der vorhergehenden Ansprüche, wobei die Füllstoffschicht (120) ein Material umfasst, dass Wärme als Reaktion auf ein elektrisches Signal erzeugt oder einen sich durch Kombination von Partikeln, die von außen eingeführt werden, ändernden Widerstand aufweist, und optional wobei die Füllstoffschicht mindestens einen von Ruß, Grafit, Metall, leitfähiges Polymer, Metallpulver, CNT, Oxide, Borid, Karbid und Chalkogenide umfasst.

11. Elektronische Vorrichtung (20, 30, 40), umfassend eine Füllstoffstruktur nach einem der vorhergehenden Ansprüche.

12. Elektronische Vorrichtung nach Anspruch 11, wobei das Substrat einen Hohlraum (C) umfasst.

13. Elektronische Vorrichtung nach Anspruch 11 oder 12, wobei ein in der Isolierschicht (140) enthaltener Alkalioxid-Inhaltsanteil geringer ist als ein in der Schutzschicht (150) enthaltener Alkalioxid-Inhaltsanteil.

14. Elektronische Vorrichtung (30) nach einem der Ansprüche 11 bis 13, wobei die Füllstoffschicht (314) Licht durch Erzeugen von Wärme emittiert.

15. Elektronische Vorrichtung (30) nach Anspruch 14, ferner umfassend:
eine Gaskammer (330), durch welche Gas eingeführt wird; und
einen Fotodetektor (340), der konfiguriert ist, um Licht, das die Gaskammer durchläuft, im durch die Füllstoffschicht emittierten Licht nachzuweisen.

## Revendications

1. Structure de charge comprenant :
un substrat (110) ;
une couche de charge (120) espacée du substrat, comprenant une couche de matériau formant matrice et une pluralité de charges conductrices et configurée pour générer de la chaleur en réponse à un signal électrique ou pour avoir un changement de résistance par combinaison de particules introduites de l'extérieur ;
une électrode (130) configurée pour être en contact avec la couche de charge et fournir un signal électrique à la couche de charge ;
et une couche isolante (140) agencée entre le substrat et l'électrode, ayant un oxyde alcalin à une teneur de 7 % ou moins et configurée pour empêcher qu'un courant de fuite soit généré entre le substrat (110) et les électrodes (130) ou entre le substrat (110) et la couche de charge (120).

2. Structure de charge selon la revendication 1, dans laquelle au moins l'une de la couche isolante et de la couche de matériau formant matrice comprend une fritte de verre.

3. Structure de charge selon la revendication 2, dans laquelle la fritte de verre comprend une fritte de verre d'oxyde.

4. Structure de charge selon la revendication 2 ou 3, dans laquelle une température de transition vitreuse de la couche isolante est 400 °C ou plus.

5. Structure de charge selon l'une quelconque des revendications précédentes, comprenant en outre : une couche de protection (150) configurée pour être en contact avec le substrat (110) et empêcher l'oxydation du substrat.

6. Structure de charge selon la revendication 5, dans laquelle au moins une partie d'un matériau inclus dans la couche isolante (140) est la même qu'un matériau inclus dans la couche de protection (150), et optionnellement dans laquelle des matériaux couramment inclus dans la couche isolante et la couche de protection ont des teneurs différentes.

7. Structure de charge selon l'une quelconque des revendications précédentes, dans laquelle un coefficient de dilatation thermique de la couche isolante va de 5 (µm/m/°C) à 12 (µm/m/°C).

8. Structure de charge selon l'une quelconque des revendications précédentes, dans laquelle l'électrode (130) est agencée entre la couche de charge et le substrat.

9. Structure de charge selon l'une quelconque des revendications précédentes, dans laquelle la couche de charge (120) est agencée entre l'électrode (130) et le substrat (110).

10. Structure de charge selon l'une quelconque des revendications précédentes, dans laquelle la couche de charge (120) comprend un matériau qui génère de la chaleur en réponse à un signal électrique ou a un changement de résistance par combinaison de particules introduites de l'extérieur et, optionnellement, dans laquelle la couche de charge comprend au moins l'un de : noir de carbone, graphite, métal, polymère conducteur, poudre métallique, CNT, oxyde, borure, carbure, et chalcogénure.

11. Dispositif électronique (20, 30, 40) comprenant une structure de charge selon l'une quelconque des revendications précédentes.

12. Dispositif électronique selon la revendication 11, dans lequel le substrat comprend une cavité (C).

13. Dispositif électronique selon la revendication 11 ou 12, dans lequel une teneur en oxyde alcalin de la couche isolante (140) est inférieure à la teneur en oxyde alcalin de la couche de protection (150).

14. Dispositif électronique (30) selon l'une quelconque des revendications 11 à 13, dans lequel la couche de charge (314) émet une lumière en générant de la chaleur.

15. Dispositif électronique (30) selon la revendication 14, comprenant en outre :
une chambre à gaz (330) par laquelle est introduit un gaz, et
un photodétecteur (340) configuré pour détecter la lumière traversant la chambre à gaz dans la lumière émise par la couche de charge.
